# EUROPEAN PATENT SPECIFICATION

(11) **EP 3 703 074 B1**
(45) Date of publication and mention of the grant of the patent: **15.05.2024**
(21) Application number: 20157521.4
(22) Date of filing: 14.02.2020
(51) Int. Cl.: G16H 40/63

(54) **PATIENT BED WITH INTERFACE FOR MANUAL INPUT OF ITS LOCATION**
PATIENTENBETT MIT SCHNITTSTELLE FÜR DIE MANUELLE EINGABE SEINES STANDORTES
LIT DU PATIENT AVEC INTERFACE POUR LA SAISIE MANUELLE DE SON EMPLACEMENT

(30) Priority: 26.02.2019 US 201962810445 P
(43) Date of publication of application: 02.09.2020
(73) Proprietor: Hill-Rom Services, Inc., Batesville, IN 47006-9167 (US)
(72) Inventor: HEIMBROCK, Richard H, Batesville, IN 47006-9167 (US); GUNN, Jennifer A, Batesville, IN 47006-9167 (US); SCHRODER, John S, Batesville, IN 47006-9167 (US)
(74) Representative: Findlay, Alice Rosemary

(56) References cited:
- US-A1- 2018 039 743

## Description

The present disclosure relates to patient beds and particularly, to patient beds having equipment used in connection with determining locations of the patient beds in a healthcare facility. More particularly, the present disclosure relates to patient beds having bed interfaces for manual location of the patient beds.

Some healthcare facilities, such as hospitals, nursing homes, and the like, have nurse call systems that receive bed status data via a wired connection to a patient bed. For example, the NaviCare^{®} Nurse Call system available from Hill-Rom Company, Inc. uses a 37-pin cable to interconnect patient beds to a bed interface unit (BIU) or network interface unit (NIU) or audio station bed connector (ASBC). The BIU's, NIU's, and ASBC's have identification (ID) codes such as serial numbers and/or MAC addresses that can be correlated with a room location in the healthcare facility. Thus, by connecting the patient beds to the respective BIU's, NIU's, or ASBC's, as the case may be, a remote computer device such as a server of the nurse call system is able to receive bed ID data and location ID data and determine the room locations of the various beds in the healthcare facility.

However, not all healthcare facilities have nurse call systems to which patient beds couple via a wired connection. In recent times, some patient beds are equipped with wireless communication circuitry for WiFi communication between the patient beds and wireless access points (WAP's) of the healthcare facility. The radio frequency (RF) signals from the WiFi circuitry of the patient beds is able to pass through walls, floors, and ceilings such that multiple WAP's may receive the RF signals transmitted from the beds. Accordingly, the room locations of such beds cannot be determined with absolute certainty based on which WAP's are able to communicate with the beds.

US 20180039743A1 discloses systems and methods of associating beds and/or rooms and/or patients. One of the disclosed systems and methods involves manually entering location information on a graphical user interface of a patient bed for subsequent transmission after a bed has been plugged into AC an power outlet.

The invention is set out by the appended claims.

According to a first aspect of the present disclosure, a patient bed includes a frame that is configured to support a patient, circuitry that is carried by the frame, and a graphical user interface (GUI) that is carried by the frame and that is coupled to the circuitry. The graphical user interface displays at least one user interface screen that is used by a caregiver to manually enter location data that is indicative of a location in a healthcare facility at which the patient bed is located. The circuitry is configured to transmit the location data entered by the caregiver and a bed identification (ID) from the bed. The circuitry commands the GUI to display a first screen of the at least one interface screen that is used by the caregiver to manually enter location data. The first screen is displayed after a threshold period of time has elapsed subsequent to a power plug of the circuitry being plugged into an alternating current (AC) outlet of the healthcare facility and subsequent to casters of the frame being braked.

In some embodiments of the first aspect, the location data may include a room number of the healthcare facility. If desired, the GUI of the first aspect may include a change location button that may be selectable by the caregiver to initiate a change of the location data prior to the threshold period of time elapsing. A room menu screen may appear on the GUI in response to the change location button being selected. The room menu screen may permit the caregiver to select a room number from a list of room numbers. Alternatively or additionally, a campus icon and a unit icon may appear on the GUI in response to the change location button being selected. The campus icon may be selectable to cause a campus menu list to be displayed on the GUI and the unit icon may be selectable to cause a unit menu list to be displayed on the GUI.

The first screen appearing on the GUI after the threshold period of time may have elapsed may include a button that may be selectable to initiate manual entry of the location data. This button on the first screen is different than the change location button mentioned above. However, a room menu screen may appear on the GUI in response to the button being selected on the first screen. The room menu screen may permit the caregiver to select a room number from a list of room numbers. Alternatively or additionally, a campus icon and a unit icon may appears on the GUI in response to the button being selected on the first screen. The campus icon may be selectable to cause a campus menu list to be displayed on the GUI and the unit icon may be selectable to cause a unit menu list to be displayed on the GUI.

In some embodiments of the first aspect, the circuitry may include a wireless communication module that may be configured to wirelessly transmit the location data and the bed ID to a wireless access point for delivery to at least one remote computer for purposes of making a bed-to-room association. Optionally, the wireless communication module also may be configured to transmit bed status data from the bed. If desired, the circuitry may be configured to play a voice prompt to remind the caregiver to manually enter the location data after the threshold period of time may have elapsed.

According to a second aspect of the present disclosure, a patient bed includes a frame that is configured to support a patient, circuitry that is carried by the frame, and a graphical user interface (GUI) that is carried by the frame and that is coupled to the circuitry. The graphical user interface displays at least one user interface screen that is used by a caregiver to manually enter location data that is indicative of a location in a healthcare facility at which the patient bed is located. The circuitry is configured to transmit the location data entered by the caregiver and a bed identification (ID) from the bed. The circuitry is configured to play a voice prompt to remind the caregiver to manually enter the location data after a threshold period of time has elapsed subsequent to a power plug of the circuitry being plugged into an alternating current (AC) outlet of the healthcare facility and subsequent to casters of the frame being braked.

In some embodiments of the second aspect, the circuitry also may command the GUI to display a first screen of at least one interface screen that may be used by the caregiver to manually enter location data after the threshold period of time may have elapsed. The location data may include a room number, for example. Optionally, the first screen appearing on the GUI may include a button that is selectable to initiate manual entry of the location data. If desired, a room menu screen may appear on the GUI in response to the button being selected on the first screen. The room menu screen may permit the caregiver to select a room number from a list of room numbers. Alternatively or additionally, a campus icon and a unit icon may appear on the GUI in response to the button being selected on the first screen. The campus icon may be selectable to cause a campus menu list to be displayed on the GUI and the unit icon may be selectable to cause a unit menu list to be displayed on the GUI.

Further according to the second aspect, the circuitry may include a wireless communication module that may be configured to wirelessly transmit the location data and the bed ID to a wireless access point for delivery to at least one remote computer for purposes of making a bed-to-room association. Optionally, the wireless communication module also may transmit bed status data from the bed.

In some embodiments of the second aspect, the GUI may include a change location button that may be selectable by the caregiver to initiate a change of the location data prior to the threshold period of time elapsing. A room menu screen may appear on the GUI in response to the change location button being selected. The room menu screen may permit the caregiver to select a room number from a list of room numbers. Alternatively or additionally, a campus icon and a unit icon may appear on the GUI in response to the change location button being selected. The campus icon may be selectable to cause a campus menu list to be displayed on the GUI and the unit icon may be selectable to cause a unit menu list to be displayed on the GUI.

The invention at least in preferred embodiments provides an inexpensive way to determine the locations of patient beds having wireless communication capability but that are not coupled to nurse call systems via wired connections.

The invention will now be further described by way of example with reference to the accompanying drawings, in which:
Fig. 1 is a diagrammatic view of a bed manual locating system showing a patient bed wirelessly coupled to a network via a wireless access point for communication with a real time locating system (RTLS) server, an electronic medical record (EMR) server, and a communications server;
Fig. 2 is a block diagram showing various components of the patient bed of Fig. 1 and also showing the RTLS, EMR, nurse call, and communications systems of the network;
Fig. 3 is a diagrammatic view showing a graphical user interface (GUI) of the patient bed having a touch screen display that displays a window having a room number and an edit icon or button; and
Figs. 4A and 4B together make up a diagrammatic view having a flow chart portion in Fig. 4A and having screen flows showing how the GUI of the bed is used to manually enter location data for the patient bed.

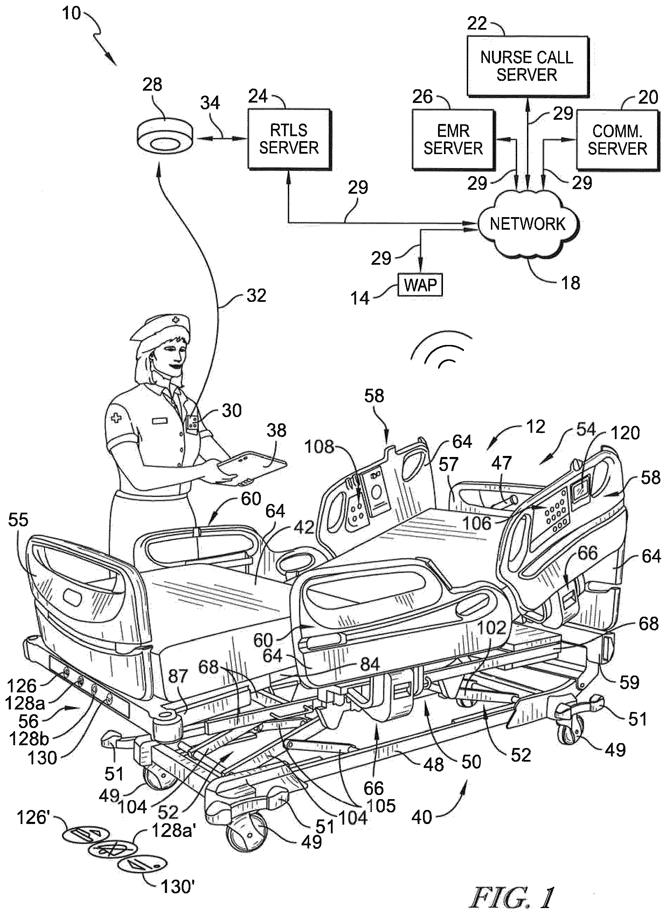

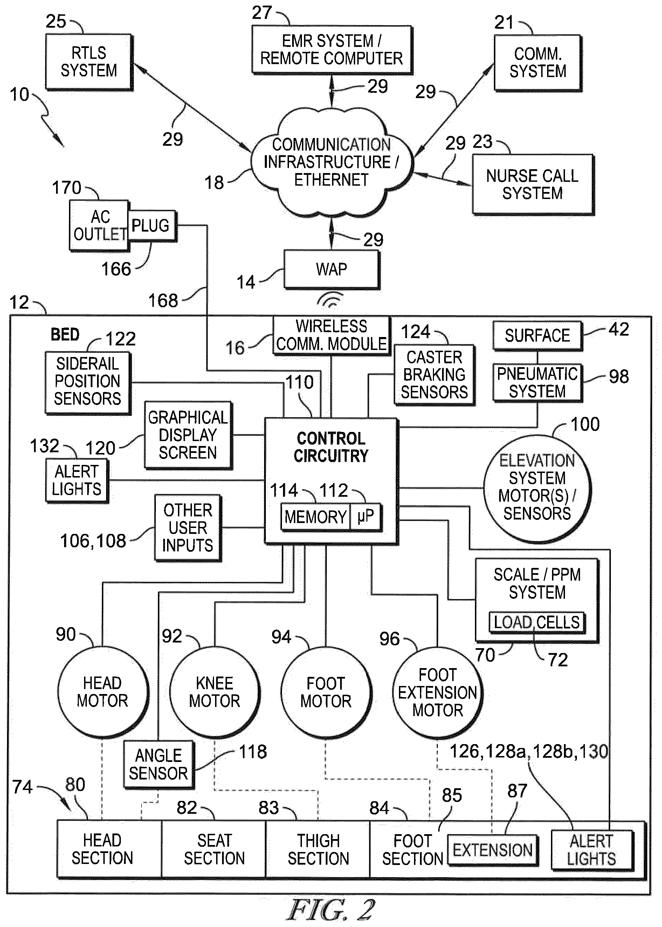

A bed manual locating system 10 includes a plurality of patient beds 12, only one of which is shown in Fig. 1, coupled to a network 18 of a healthcare facility such as a hospital, outpatient care facility, nursing home, and the like. In the illustrative example, patient bed 12 is coupled to network 18 via a wireless access point (WAP) 14 which may or may not be located in the respective patient room with the bed 12. It should be understood that, although only one WAP 14 is shown in Fig. 1, multiple WAP's 14 may be in wireless communication with bed 12. Illustratively, bed 12 includes a wireless communication module 16, as shown in Fig. 2, that sends wireless signals, such as radio frequency (RF) signals, or transmissions to WAP 14 and that receives wireless signals, such as RF signals, or transmissions from WAP. Thus, wireless communication module 16 includes a transceiver in some embodiments.

As shown diagrammatically in Figs. 1 and 2, a communication server 20 of a corresponding communication system 21, a nurse call server 22 of a corresponding nurse call system 23, a real time locating system (RLTS) server 24 of a corresponding RTLS 25, and an electronic medical records (EMR) server 26 of an EMR system 27 are each coupled to network 18. Block 27 also is intended to represent other remote computers of network 18. Double headed arrows 29 in Figs. 1 and 2 represent the bidirectional communication links between network 18, WAP 14 (and the corresponding bed 12) and each of servers 20, 22, 24, 27 of respective systems 21, 23, 25, 27 and therefore, with each other. Communication links 29 include wired communication links or wireless communication links or both at the option of the designer of system 10 in any given healthcare facility. The wireless communication between the WAP's 14 of network 18 and module 16 of bed 12 contemplated by this disclosure includes Bluetooth (BT), Bluetooth Low Energy (BLE), Zigbee, Z-Wave, and WiFi (e.g., any of the 802.11ₓ protocols). However, this is not rule out other types of wireless communication between bed 12 and an appropriately configured transceiver in addition to or in lieu of WAP 14, including infrared (IR) communications, ultrasonic (US) communications, ultra-wideband (UWB) communications, and so forth.

The RTLS 25 of system 10 includes wireless transceiver units 28 placed throughout the healthcare facility. Only one such unit 28 is depicted diagrammatically in Fig. 1. The RTLS 25 of system 10 also includes caregiver locating or tracking tags or badges 30 that are worn by caregivers. Each of the transceiver units 28 receives a wireless signal from the badges 30 of each of the caregivers wearing badges 30 and that are within communication range of the respective unit 28 as indicated diagrammatically by arrow 32 in Fig. 1. The wireless signal from each badge 30 includes badge identification data (ID) which is unique to the corresponding badge 30. Unit 28 then transmits its ID, which corresponds to a particular location in the healthcare facility, and the badge (ID) to RTLS server 24 as indicated diagrammatically by a bidirectional communication link 34 in Fig. 1. Based on the received badge ID and the location ID from unit 28, server 24 determines the location of the caregiver within the healthcare facility. Some healthcare facilities do not include RTLS 25 in which case server 24, transceivers 28, and badges 30 are omitted from system 10.

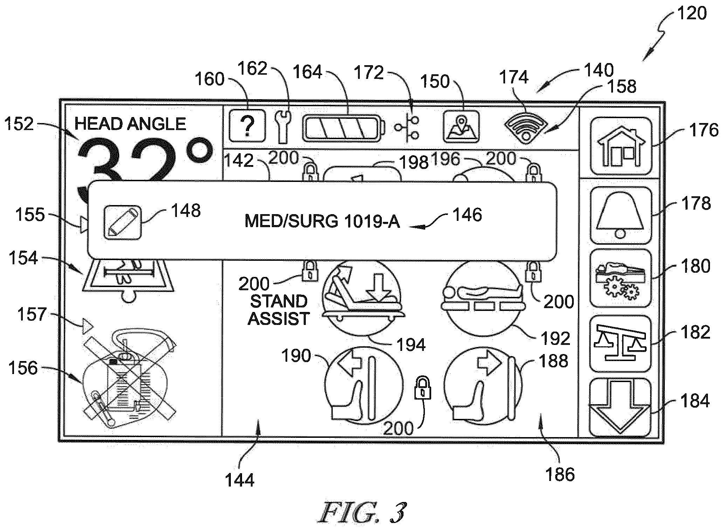

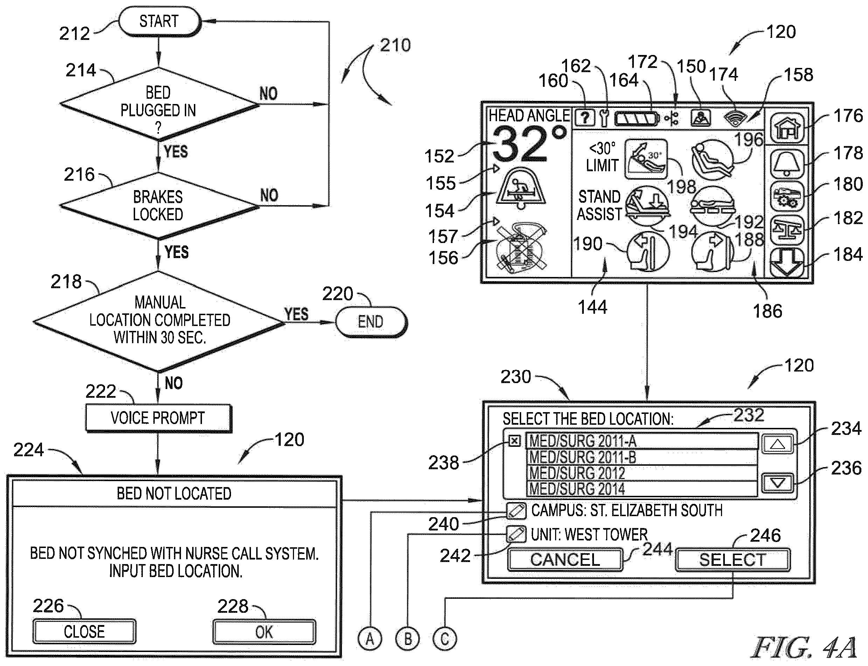

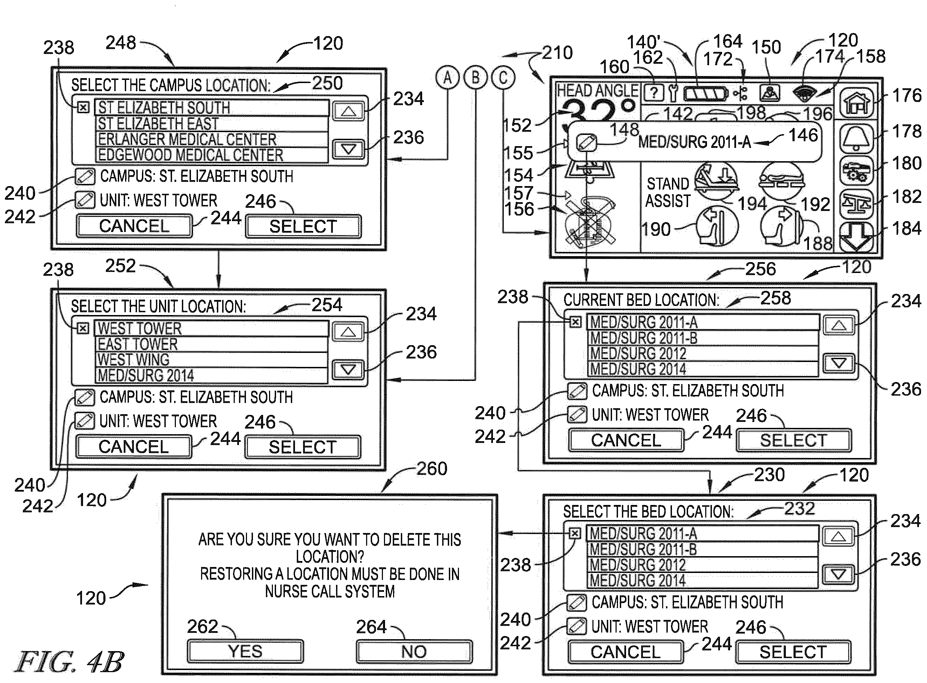

In some embodiments, RTLS server 24 is included in system 10 and transceivers 28 and badges 30 are omitted. In such embodiments, RTLS server 24 serves as a data repository for bed-to-room associations that are established via manual entry at beds 12 as will be discussed in further detail below in connection with Figs. 3, 4A and 4B. Alternatively, the bed-to-room associations established via manual entry at beds 12 are stored in another server, such as nurse call server 22, EMR server, or any other server of system 10, including severs 20. Thus, servers 20, 22, 26, or other servers of system 10, may serve multiple purposes and so these may be configured to serve the RTLS function along with their other function(s) (e.g., nurse call, EMR, communications, etc.).

In some embodiments having RTLS 25 with transceivers 28 and tags 30 to track caregiver locations, the location of assets such as beds 12 can also be tracked in a similar manner by server 24 by attaching asset tags that are substantially the same as badges 30 to the assets to be tracked. However, in such systems 10 in which beds 12 have asset tags for tracking by RTLS 25, then manual association of beds 12 to rooms as discussed below in connection with Figs. 3, 4A and 4B is not needed. However, hybrid systems 10 in which some locations of beds 12 are determined by signals from respective asset tags attached to the beds 12 and in which some locations of beds 12 are determined by manual entry at the respective beds 12, are contemplated by the present disclosure. In Fig. 1, the caregiver is shown carrying a tablet computer 38 which is configured to communicate wirelessly with other devices of network 18 such as via communications server 20. Mobile phones are carried by caregivers in addition to, or in lieu of tablet computers 38, and are in communication with communication server 20 in some embodiments.

As alluded to above, the present disclosure is primarily focused on manual entry of locating information at beds 12. However, a discussion is provided below of the basic components and operation of various features of bed 12 so that an understanding of the types of bed status data transmitted wirelessly by module 16 to WAP 14 can be gained. From the below discussion it will be appreciate that beds 12 having capability for manual entry of location information are by themselves, or in combination with the other components of system 10, a practical application of the concepts and ideas disclosed herein. Bed 12 includes a patient support structure such as a frame 40 that supports a surface or mattress 42 as shown in Fig. 1. It should be understood that Fig. 1 shows some details of one possible bed 12 having one specific configuration. In particular, illustrative bed 12 of Fig. 1 is the CENTRELLA^{®} bed available from Hill-Rom Company, Inc. Other aspects of illustrative bed 12 are shown and described in more detail in U.S. Patent Application Publication No. 2018/0161225 A1. However, this disclosure is applicable to other types of patient support apparatuses 12 having other configurations, including other types of beds, surgical tables, examination tables, stretchers, chairs, wheelchairs, patient lifts and the like.

Still referring to Fig. 1, frame 40 of bed 12 includes a base frame 48 (sometimes just referred herein to as a base 48), an upper frame assembly 50 and a lift system 52 coupling upper frame assembly 50 to base 48. Lift system 52 is operable to raise, lower, and tilt upper frame assembly 50 relative to base 48. Bed 12 has a head end 54 and a foot end 56. Patient bed 12 further includes a footboard 55 at the foot end 56 and a headboard 57 at the head end 54. Illustrative bed 12 includes a pair of push handles 47 coupled to an upstanding portion 59 of base 48 at the head end 54 of bed 12. Only a portion of one push handle 47 can be seen in Fig. 1. Headboard 57 is coupled to upstanding portion 59 of base as well. Footboard 55 is coupled to upper frame assembly 50. Base 48 includes wheels or casters 49 that roll along floor (not shown) as bed 12 is moved from one location to another. A set of foot pedals 51 are coupled to base 48 and are used to brake and release casters 49.

Illustrative patient bed 12 has four siderail assemblies coupled to upper frame assembly 50 as shown in Fig. 1. The four siderail assemblies include a pair of head siderail assemblies 58 (sometimes referred to as head rails) and a pair of foot siderail assemblies 60 (sometimes referred to as foot rails). Each of the siderail assemblies 58 and 60 is movable between a raised position, as shown in Fig. 1, and a lowered position (not shown). Siderail assemblies 58, 60 are sometimes referred to herein as siderails 58, 60. Each siderail 58, 60 includes a barrier panel 64 and a linkage 66. Each linkage 66 is coupled to the upper frame assembly 50 and is configured to guide the barrier panel 64 during movement of siderails 58, 60 between the respective raised and lowered positions. Barrier panel 64 is maintained by the linkage 66 in a substantially vertical orientation during movement of siderails 58, 60 between the respective raised and lowered positions.

Upper frame assembly 50 includes various frame elements 68, shown in Fig. 1, that form, for example, a lift frame and a weigh frame supported with respect to the lift frame by a set of load cells 72 of a scale and/or bed exit/patient position monitoring (PPM) system 70 of bed 12, as shown diagrammatically in Fig. 2. A patient support deck 74, shown diagrammatically in Fig. 2, is carried by the weigh frame portion of upper frame assembly 50 and supports mattress 42 thereon. Data relating to the operation of the scale and/or bed exit/PPM system 70 is among the features of bed 12 for which bed status data is transmitted wirelessly from module 16 to one or more WAP's 14.

Patient support deck 74 includes a head section 80, a seat section 82, a thigh section 83 and a foot section 84 in the illustrative example as shown diagrammatically in Fig. 2. Sections 80, 83, 84 are each movable relative to the weigh frame portion of upper frame assembly 50. For example, head section 80 pivotably raises and lowers relative to seat section 82 whereas foot section 84 pivotably raises and lowers relative to thigh section 83. Additionally, thigh section 83 articulates relative to seat section 82. Also, in some embodiments, foot section 84 is extendable and retractable to change the overall length of foot section 84 and therefore, to change the overall length of deck 74. For example, foot section 84 includes a main portion 85 and an extension 87 in some embodiments as shown diagrammatically in Fig. 2.

In the illustrative embodiment, seat section 82 is fixed in position with respect to the weigh frame portion of upper frame assembly 50 as patient support deck 74 moves between its various patient supporting positions including a horizontal position to support the patient in a supine position, for example, and a chair position (not shown) to support the patient in a sitting up position. In other embodiments, seat section 82 also moves relative to upper frame assembly 50, such as by pivoting and/or translating. Of course, in those embodiments in which seat section 82 translates relative to the upper frame assembly 50, the thigh and foot sections 83, 84 also translate along with seat section 82. As bed 12 moves from the horizontal position to the chair position, foot section 84 lowers relative to thigh section 83 and shortens in length due to retraction of the extension 87 relative to main portion 85. As bed 12 moves from the chair position to the horizontal position, foot section 84 raises relative to thigh section 83 and increases in length due to extension of the extension 87 relative to main portion 85. Thus, in the chair position, head section 80 extends upwardly from upper frame assembly 50 and foot section 84 extends downwardly from thigh section 83.

As shown diagrammatically in Fig. 2, bed 12 includes a head motor or actuator 90 coupled to head section 80, a knee motor or actuator 92 coupled to thigh section 83, a foot motor or actuator 94 coupled to foot section 84, and a foot extension motor or actuator 96 coupled to foot extension 87. Motors 90, 92, 94, 96 may include, for example, an electric motor of a linear actuator. In those embodiments in which seat section 82 translates along upper frame assembly 50 as mentioned above, a seat motor or actuator (not shown) is also provided. Head motor 90 is operable to raise and lower head section 80, knee motor 92 is operable to articulate thigh section 83 relative to seat section 82, foot motor 94 is operable to raise and lower foot section 84 relative to thigh section 83, and foot extension motor 96 is operable to extend and retract extension 87 of foot section 84 relative to main portion 85 of foot section 84. Data relating to the operation of motors 90, 92, 94, 96 and the positions of deck sections 80, 82, 83, 84 is among the features of bed 12 for which bed status data is transmitted wirelessly from module 16 to one or more WAP's 14.

In some embodiments, bed 12 includes a pneumatic system 98 that controls inflation and deflation of various air bladders or cells of mattress 42. The pneumatic system 98 is represented in Fig. 2 as a single block but that block 98 is intended to represent one or more air sources (e.g., a fan, a blower, a compressor) and associated valves, manifolds, air passages, air lines or tubes, pressure sensors, and the like, as well as the associated electric circuitry, that are typically included in a pneumatic system for inflating and deflating air bladders of mattresses. Operation of pneumatic system 98 is among the features of bed 12 for which bed status data is transmitted wirelessly from module 16 to one or more WAP's 14.

As also shown diagrammatically in Fig. 2, lift system 52 of bed 10 includes one or more elevation system motors or actuators 100, which in some embodiments, comprise linear actuators with electric motors. Thus, actuators 100 are sometimes referred to herein as motors 100 and operation of the motors 100 is among the features of bed 12 for which bed status data is transmitted wirelessly from module 16 to one or more WAP's. Alternative actuators or motors contemplated by this disclosure include hydraulic cylinders and pneumatic cylinders, for example. The motors 100 of lift system 52 are operable to raise, lower, and tilt upper frame assembly 50 relative to base 48. In the illustrative embodiment, one of motors 100 is coupled to, and acts upon, a set of head end lift arms 102 and another of motors 100 is coupled to, and acts upon, a set of foot end lift arms 104 to accomplish the raising, lowering and tilting functions of upper frame 50 relative to base 48. Guide links 105 are coupled to base 48 and to lift arms 104 in the illustrative example as shown in Fig. 1.

Each of siderails 58 includes a first user control panel 106 coupled to the outward side of the associated barrier panel 64. Controls panels 106 include various buttons that are used by a caregiver to control associated functions of bed 12. For example, control panel 106 includes buttons that are used to operate head motor 90 to raise and lower the head section 80, buttons that are used to operate knee motor 92 to raise and lower the thigh section 83, and buttons that are used to operate motors 100 to raise, lower, and tilt upper frame assembly 50 relative to base 48. In some embodiments, control panel 106 also includes buttons that are used to operate motor 94 to raise and lower foot section 84 and buttons that are used to operate motor 96 to extend and retract foot extension 87 relative to main portion 85. Each of siderails 58 also includes a second user control panel 108 coupled to the inward side of the associated barrier panel 64. Controls panels 108 include various buttons that are used by a patient to control associated functions of bed 12. In some embodiments, the buttons of control panels 106, 108 comprise membrane switches that are used to control head motor 90 and knee motor 92.

As shown diagrammatically in Fig. 2, bed 12 includes control circuitry 110 that is electrically coupled to motors 90, 92, 94, 96 and to motors 100 of lift system 52. Control circuitry 110 is sometimes referred to as a "controller." Control circuitry 110 is represented diagrammatically as a single block in Fig. 2, but control circuitry 110 in some embodiments, comprises various circuit boards, electronics modules, and the like that are electrically and communicatively interconnected. Control circuitry 110 includes one or more microprocessors 112 or microcontrollers that execute software to perform the various control functions and algorithms described herein. Thus, circuitry 110 also includes memory 114 for storing software, variables, calculated values, and the like as is well known in the art. Memory 112 comprises, for example, one or more flash memory banks such as one or more EEPROM's , EPROM's, and the like. In some embodiments, memory 112 is included on the same integrated circuit chip as microprocessor 112.

As shown diagrammatically in Fig. 2, an "other user inputs" block represents the various user inputs such as buttons of control panels 106, 108, for example, that are used by the caregiver or patient to communicate input signals to control circuitry 110 of bed 12 to command the operation of the various motors 90, 92, 94, 96, 100 of bed 12, as well as commanding the operation of other functions of bed 12. Bed 12 includes at least one graphical user input (GUI) or display screen 120 coupled to a respective siderail 58 as shown in Fig. 1. Display screen 120 is coupled to control circuitry 110 as shown diagrammatically in Fig. 2. In some embodiments, two graphical user interfaces 120 are provided and are coupled to respective siderails 58. Alternatively or additionally, one or more graphical user interfaces are coupled to siderails 60 and/or to one or both of the headboard 57 and footboard 55 or some other portion of bed 12 such as a support arm assembly extending upwardly from base frame 48 or upper frame 50.

Still referring to Fig. 2, wireless communication module 16 is also coupled electrically to control circuitry 110 and is configured for wireless communication with network 18 and its associated devices without the use of any wired bed cable in the illustrative embodiment. The communication of data from module 16 is among the features of bed 12 controlled by the bed operating software of control circuitry 110.

Still referring to Fig. 2, bed 12 includes various sensors to sense the states or positions of various portions of bed 12. In the illustrative example, bed 12 includes an angle sensor 118 coupled to head section 80 to sense an angle of head section elevation (sometimes referred to as the head-of-bed (HOB) angle). Angle sensor 118 includes an accelerometer (single-axis or multi-axis) in some embodiments. In such embodiments, the HOB angle is measured with respect to a horizontal reference axis and/or with respect to a vertical reference axis depending upon the orientation of the accelerometer relative to head section 80 and depending upon the type of accelerometer used. In other embodiments, angle sensor 118 includes a rotary potentiometer which measures the HOB angle between head section 90 and another portion of frame 40 such as one of frame members 68 of upper frame assembly 50. In further embodiments, angle sensor 118 is included in head motor 90 and has an output that correlates to the HOB angle. Motor 90 may include, for example, a shaft encoder, a Hall effect sensor, a rotary potentiometer, or some other sensor which serves as angle sensor 118 of bed 12 in such embodiments. Similar such sensors are included in elevation system motors 100 in some embodiments and are used to determine the position of upper frame assembly 50 relative to base 48 such as the height of upper frame assembly 50 and/or amount of tilt of upper frame assembly 50 relative to base 48.

Bed 12 also includes siderail position sensors 122 to sense the position (e.g., raised and/or lowered) of each of siderails 58, 60 and one or more caster braking sensors 124 to sense whether casters 49 are braked or released. In some embodiments, sensors 122, 124 include limit switches that are engaged or disengaged by a linkage mechanism, such as linkage 66 in the case of siderails 58, 60, to produce output signals indicative of the position of the respective mechanical structure. Alternatively, Hall effect sensors may be used as some or all of sensors 122, 124 in some embodiments. The foregoing types of sensors 122, 124 are just a couple examples of suitable sensors and therefore, this disclosure is intended to cover all types of sensors that may be used as sensors 122, 124. Each of the sensors mentioned above, including sensors internal to motors 100 and sensors 118, 122, 124 are each coupled electrical to control circuitry 110 for analysis and/or processing. Thus, data from sensors 118, 122, 124 is used by the bed operating software in connection with the control and operation of various features of bed 12 and is among the features of bed 12 for which bed status data is transmitted wirelessly from module 16 to one or more WAP's 14.

As shown in Fig. 1, bed 12 includes four status or alert lights 126, 128a, 128b, 130 at foot end 56 corresponding to various monitored features of bed 12. In the illustrative embodiment, for example, bed 12 includes a siderail position light 126, a bed exit/PPM disabled light 128a, a bed exit/PPM enabled light 128b, and a bed lowest position light 130. PPM is an acronym for "patient position monitoring." Alert lights 126, 128a, 128b, 130 are coupled to a lateral frame member of extension 87 of foot section 84 and are situated beneath footboard 55. In other embodiments, alert lights 126, 128a, 128b, 130 may be located elsewhere on bed 12 such as on base 48 and/or one or more of siderails 58, 60. In Fig. 2, alert lights 126, 128a, 128b, 130 are represented diagrammatically as a single block and are coupled electrically to control circuitry 110 to control the manner in which alert lights 126, 128a, 128b, 130 are illuminated as will be discussed in further detail below. In some embodiments, other alert lights 132, shown diagrammatically as a single block in Fig. 2, are located elsewhere on bed 12, such as on siderails 58, 60, and are illuminated to convey information regarding other features of bed 12, such as to indicate motor lockout conditions, alarm volume control levels, nurse call status, caster brake status, and the like.

In some embodiments, alert lights 126, 128b, 130 are illuminated different colors to indicate certain statuses. For example, lights 126, 128b, 130 are illuminated a first color, such as green for example, if the associated bed condition is in an acceptable or statifcatory state. Lights 126, 128b, 130 are illuminated a second color, such as amber or yellow for example, if the associated bed condition is an undesirable or unsatisfactory state. Each of lights 126, 128a, 128b, 130 has an icon on the lens of the respective light 126, 128a, 128b, 130 corresponding to the monitored condition of bed 12.

In the illustrative example, if bed 12 has a falls risk protocol enabled (i.e., turned on) in which all of siderails 58, 60 are required to be raised (or a subset of siderails 58, 60 selected on GUI 120 is required to be raised), the light 126 is illuminated green if all of the siderails 58, 60 (or selected subset of siderails 58, 60) are in the respective raised positions (e.g., the desirable or satisfactory condition) and the light 126 is illuminated amber, and in some embodiments flashed, if any one or more of siderails 58, 60 (or selected subset of siderails 58, 60) is in the lowered position (e.g., the undesirable or unsatisfactory condition). In some embodiments, a lighted iconic image 126' corresponding to the state of light 126 is projected onto the floor at the foot end of the bed 12 as shown in Fig. 1. Image 126' has the same color and icon as light 126. If the falls risk protocol of bed 12 is disabled (i.e., turned off), then light 126 is turned off and no image 126' is projected onto the floor by bed 12.

If the bed exit/PPM system of bed 12 is disabled (i.e., turned off), then light 128a is illuminated blue and a corresponding blue lighted iconic image 128a' is projected onto the floor by bed 12. If the bed exit/PPM system of bed 12 is enabled (i.e., turned on), then light 128b is illuminated and a corresponding lighted iconic image (not shown) is projected onto the floor by bed 12 and appears in the same general location as image 128a'. Of course, when light 128b is illuminated, light 128a is turned off and image 128a' is no longer projected onto the floor. Light 128b and image 128b' are illuminated green when the bed exit/PPM system is armed (aka enabled) and the patient is on the bed in the proper location (e.g., the desirable or satisfactory condition). Some embodiments of bed 12 have multiple modes (e.g., patient movement, pre-exit, and exiting modes) with varying levels of sensitivities at which an alarm condition is considered to exist. Light 128b and image 128b' are illuminated amber, and in some embodiments are flashed, if the bed exit/PPM system is armed and the patient is not properly positioned on bed, including being out of bed altogether (e.g., the undesirable or unsatisfactory condition).

If bed 12 has the falls risk protocol enabled (i.e., turned on) control circuitry 110 monitors the position of the upper frame 50 relative to base frame 48 to assure that upper frame 50 is in its lowest position relative to base frame 48. If upper frame 50 is in its lowest position (e.g., the desirable or satisfactory condition), the light 130 is illuminated green (e.g., the desirable or satisfactory condition). On the other hand, if upper frame 50 is not in its lowest position (e.g., the undesirable or unsatisfactory condition), the light 130 is illuminated amber, and in some embodiments flashed. In some embodiments, a lighted iconic image 130' corresponding to the state of light 130 is projected onto the floor at the foot end of the bed 12 as shown in Fig. 1. Image 130' has the same color and icon as light 130. If the falls risk protocol of bed 12 is disabled (i.e., turned off), then light 130 is turned off and no image 130' is projected onto the floor by bed 12.

In some embodiments, an audible alarm of bed 12 may also sound under the control of control circuitry 110 if an unsatisfactory condition of a particular protocol or condition is detected. Lights 126, 128b, 130 are illuminated a third color if the associated protocol or condition is enabled for monitoring and at least one of the monitored bed statuses for the particular protocol or condition is undesirable (i.e., violated), but the associated alert has been suspended by the caregiver. If the alert has been suspended, any associated audible alarms may be turned off during the alarm suspension. A caregiver may suspend an alert associated with lights 126, 128b, 130, for example, when assisting a patient in getting out of bed 12 and going to the bathroom. The various alert conditions (aka alarm conditions) associated with the operation of alert lights 126, 128a, 126b, 130 and the audible alarms, if any, of bed 12 is among the features of bed 12 for which bed status data is transmitted wirelessly from module 16 to one or more WAP's 14.

Referring now to Fig. 3, a start-up screen 140 is shown on GUI 120. GUI 120 is embodied as a touch screen display having various icons or buttons that are selectable by a caregiver to navigate to other screens for selection of various bed functions and entry of various types of data as will be discussed below, primarily in connection with manual entry of bed location data on GUI 120. Start-up screen 140 appears on GUI, in some embodiments, in response to a caregiver (or really, any user for that matter) touching GUI 120 to wake GUI 120 from a dormant state in which GUI 120 is blacked out or, in some embodiments, in which GUI 120 displays a screen saver image of some sort.

Illustrative start-up screen 140 has a bed location window 142 superimposed over a home screen 144. Thus, Fig. 3 illustrates the situation in which a text string 146 of bed location information, illustratively "MED/SURG 1019-A," has been input manually during a previous use of GUI 120 by a caregiver. Window 142 includes an edit icon 148 that is selectable, such as by touching, to navigate to the screens that are used to edit the bed location information for whatever reason, such as if the bed 12 has been moved to a new room or if the hospital updates its room location naming convention. These edit screens are discussed below in connection with Figs. 4A and 4B. Window 142 only appears over home screen 144 for a short period of time upon startup of GUI 120, such as on the order of 5 to 30 seconds, and then disappears such that only home screen 144 remains displayed on GUI 120. In some embodiments in which a healthcare facility is not equipped to receive information wirelessly from bed 12, then the manual location entry feature using GUI 120 is disabled. In such situations, window 142 does not appear over home screen 144 upon startup of the GUI 120.

Home screen 144 includes a location icon 150 that is color coded to indicate whether bed location information or data has been entered manually using GUI 120 or, in some embodiments, if bed location data is otherwise transmitted to bed 12 either wirelessly or via a wired cable such as if bed 12 has an asset tag for locating as discussed above and RTLS server 24 sends a message to bed 12 regarding its location or if RTLS system 25 is otherwise configured to accurately determine the location of bed 12 without the use of an asset tag (e.g., the bed 12 is coupled via a cable to an NIU, BIU, or ASBC having a location ID indicative of room location). In other words, bed 12 is configured to permit bed location information to be entered manually using GUI 120 and to receive bed location information from equipment external to bed 12, in some embodiments. In any event, if bed 12 has bed location information stored in memory 114 of control circuitry 110, then bed location icon 150 is color coded green in some embodiments. If bed 12 does not have any bed location information stored in memory 114, or if bed 12 has bed location information stored in memory 114 that is believed to be inaccurate based on sensed bed conditions such as unplugging of bed 12 from a wall outlet, unbraking or releasing of casters 49, and/or movement of bed to a new location, then bed location icon 150 is color coded yellow or amber in some embodiments.

For the sake of completeness, as shown in Fig. 3, other illustrative icons and information displayed on home screen 144 includes, along a left side of home screen 144, a head angle reading 152 corresponding to the HOB angle measured by angle sensor 118, a bed exit/PPM icon 154 indicating the mode in which the scale/PPM system 70 is operating when enabled, and a urinary drainage bag lockout icon 156 indicating whether a urinary drainage bag (aka a Foley bag) is coupled to a bracket (not shown) attached to foot section 84 of bed 12. A triangular edit button 155 is provided adjacent to icon 154 and is selectable to navigate to other screens for disabling the PPM system 70 of bed 12 or changing the mode in which the PPM system 70 operates. A similar triangular edit icon 157 is provided adjacent to icon 156 and is selectable to navigate to other screens for enabling and disabling the urinary drainage bag lockout feature of bed 12. In this regard, see U.S. Patent Application Publication No. 2018/0185222 A1.

Home screen 144 includes an upper field 158 in which bed location icon 150 is shown. Upper field 158 also includes a help icon 160 that is selectable to navigate to various help screens of bed 12, a service required icon 162 that is illuminated yellow or amber and flashes when bed service is needed, and a battery charge level indicator 164 having four segments that are illuminated green to indicate a battery charge level of a battery (not shown) of bed 12. However, when the battery charge gets low, the segment to the left of indicator 164 turns yellow and flashes. The battery of bed 12 is used to supply power to various components of bed 12 when a power plug 166 at the end of a power cable 168 of bed 12 (see Fig. 2) is unplugged from an alternating current (AC) outlet 170 of the healthcare facility.

Upper field 158 further includes a nurse call connectivity icon or indicator 172 that is color coded to indicate whether bed 12 is in communication with nurse call system 23 of the healthcare facility. In some embodiments, indicator 172 is color coded white when bed 12 is not in communication with nurse call system 23 which includes embodiments of system 10 in which nurse call system 23 is omitted altogether. In some embodiments, indicator is color coded green, such as by illuminating a green circle around the indicator 172 shown in Fig. 3. Finally, in the illustrative example, upper field 158 includes a WiFi connectivity icon or indicator 174 that is color coded to indicate whether wireless communication module 16 of bed 12 is communicating with one or more WAP's 14 and, in some embodiments, indicator 174 is a received signal strength indicator (RSSI) that indicates a relative level (e.g., low, medium, high) of the received signal strength of the wirless communication from the one or more WAP's received by module 16 of bed. In this regard, segments of indicator 174 are illuminated green when wireless communications exist between module 16 and at least one WAP 14 and indicator 174 is illuminated yellow or is not illuminated at all when no wireless communications exist between module 16 and WAP 14.

Still referring to Fig. 3, along a right side of home screen 144 a menu of icons are shown including a home button 176 that is selected to return to home screen 144 when viewing a screen on GUI other than home screen 144, an alerts button 178 that is pressed to navigate to other screens for controlling alerting functions of bed 12, a mattress control button 180 that is selected to navigate to other screens for controlling functions of mattress 42 and pneumatic system 98 of bed 12, a scale control button 182 that is selected to navigate to other screens for controlling functions of scale system 70 of bed 12, and a down arrow button 184 that is selected to cause other icons (aka buttons) to appear in the menu of icons at the right side of screen 144. After button 184 is selected, an up arrow button (similar to button 184 but with the arrow pointing upwardly instead of downwardly) appears at the top of the menu of icons. In other words, the down arrow button 184 and up arrow button, when present, provide scrolling functionality to the menu of icons of screen 144.

Home screen 144 further includes a central field 186 that includes various control icons or buttons for controlling functions of bed 12 as shown in Fig. 3. Field 186 includes a foot extension button 188 that is selected to operate foot extension motor 96 to extend extension 87 relative to main portion 85 to lengthen foot section 84. Field 186 also includes a foot retraction button 190 that is selected to operate motor 96 to retract extension 87 relative to main portion 85 to shorten foot section 84. Above button 188, field 186 includes a bed flat and level button 192 that operates one or more of motors 90, 92, 94, as needed, to move deck sections 80, 82, 83, 84 into a substantially flat or substantially coplanar orientation relative to each other and to operate one or more of the elevation system motors 100, as needed, to move the upper frame 50, and therefore the deck 74, into a substantially horizontal or substantially level orientation. The word "substantially" herein is intended to mean within plus or minus 10%, or less, of the desired condition and, at a minimum, within manufacturing tolerances of the desired condition. The movements of bed 12 associated with buttons 188, 190, 192 occur only while the respective buttons 188, 190, 192 are pressed or touched. Thus, if the caregiver disengages from any of buttons 188, 190, 192 the associated bed movement stops.

Field 186 further includes a stand assist button 194 situated above button 190 in the illustrative example. Button 194 is selected when a patient is sitting at a side of the mattress 42 of bed 12 and is getting ready to stand up from the bed. While button 194 is touched, motor 90 is operated as needed to move head section 80 to a raised position, motors 92, 94 are operated as needed to flatten or lower thigh and foot section 83, 84, motors 100 are operated as need to move the upper frame 50 to its low position relative to base frame 48, and if mattress 42 is an air mattress having its pneumatic system 98 controlled by control circuitry 110, to operate the pneumatic system 98 to increase the pressure in a seat section of the mattress 42. All of these motions and operations make it easier for the patient to sand up out of bed 12. For example, by raising the head section 80, the siderails 58 are moved into ergonomic positions for the patient grip while standing up.

Field 186 includes a chair button 196 situated above button 192. Button 196 is pressed to move deck 74, and therefore mattress 42 supported by deck 74 and the patient supported by the mattress 42, into a chair position. While button 196 is pressed, motor 90 is operated as needed to pivotably raise head section 80 relative to upper frame 50, motor 92 is operated as needed to pivotably raise thigh section 83 relative to upper frame 50, and motor 94 is operated as needed to pivotably lower foot section 94 relative to thigh section 83. Field 186 also includes a 30 degree limit control button 198 for enabling and disabling a 30 degree limit function of bed 12. When enabled, the 30 degree limit control prevents head section 80 of bed 12 from being lowered to a HOB angle less than 30 degrees. By keeping head section 80 raised above 30 degrees, some pulmonary complications such as ventilated assisted pneumonia (VAP) are inhibited from occurring. Sequential presses or touches of button 198 toggles the 30 degree limit function of bed 12 between enabled (i.e., on) and disabled (i.e., off) states.

Control panel 106 of bed 12 includes a master lockout button that, when pressed, permits any of buttons 188, 190, 192, 194, 196, 198 to be simultaneously pressed to lockout the associated function of buttons 188, 190, 192, 194, 196, 198. That is, when locked out, the respective button 188, 190, 192, 194, 196, cannot be used as an input to cause the associated bed movements. Also, when locked out, button 198 cannot be used to enable the 30 degree lockout function. In essence, when button 198 is locked out, the 30 degree limit function is locked in its disabled state. A lockout icon 200 is displayed in field 186 adjacent to the respective buttons 188, 190, 192, 194, 196, 198 that have been locked out. However, a single lockout icon 200 is shown to indicate that buttons 188, 190 related to foot section 84 extension and retraction are both been locked out together. After any of buttons 188, 190, 192, 194, 196, 198 are locked out, they are unlocked in the same manner as just described. That is, the master lockout button on control panel 106 is pressed and then the locked out button 188, 190, 192, 194, 196, 198 to be unlocked is pressed simultaneously. The master lockout button on panel 106 is a "hard" or analog button such as a membrane switch, for example, whereas the buttons of home screen 144 are "soft" buttons that are implemented in software as is known in the art.

Referring now to Figs. 4A and 4B, a software algorithm 210 that is stored in memory 114 and executed by microprocessor 112 of control circuitry 110 of bed 12 is shown. Algorithm 210 includes the various steps, conditions, and user input selections that are implemented by bed 12 as a practical application for entering manual bed location data using GUI 120 of bed 12. Algorithm 210 starts at block 212 and proceeds to block 214 to determine whether bed 12 is plugged in. Thus, at block 214 control circuitry 110 determines whether plug 166 is connected to AC outlet 170 such that current or voltage is sensed by a power circuitry module (not shown) of control circuitry 110. The power circuitry module or circuitry 110 may include, for example, one or more transformers, rectifiers, voltage converters, voltage dividers, voltage regulators, resistors, inductors, capacitors, and the like, at the discretion of the bed designer.

If it is determined at block 214 of Fig. 4A that bed 12 is not plugged in, then algorithm 210 loops back to step 212 and proceeds from there. If it is determined at block 214 that bed 12 is plugged in, then algorithm 210 proceeds to block 216 where microprocessor 112 of control circuitry 110 determines whether casters 49 of bed 12 are braked or have their brakes locked against rolling based on signals from caster braking sensors 124. If it is determined at block 216 that casters 49 are not locked, then algorithm 210 loops back to step 212 and proceeds from there.

If it is determined at block 216 of Fig. 4A that casters 49 of bed 12 are braked or locked, then algorithm 210 proceeds to block 218 at which microprocessor 112 of control circuitry 110 determines whether manual location has been completed by a caregiver using GUI 120 within 30 seconds. Thus, microprocessor 112 implements a software timer for 30 seconds to give the caregiver time to manually enter bed location data using GUI 120. In this regard, when bed 12 is plugged into AC outlet 170 and casters 49 are braked, home screen 144 appears on GUI 120 but without window 142 appearing on screen 144 because there is not yet any room location associated with bed 12, as shown in the upper right region of Fig. 4A. In some embodiments, after bed 12 is plugged into outlet 170, a reminder screen appears on GUI 120 to remind the caregiver to brake casters 49 using foot pedals 51. In any event, once home screen 144 appears on GUI 120 as shown in Fig. 4A, the caregiver is able to select location button 150 to navigate to subsequent screens for manual entry of bed location information or data as described below.

If it is determined at block 218 that manual entry of bed location information has occurred within 30 seconds, then algorithm 210 ends as indicated at block 220. In other embodiments, a time threshold greater than or less than 30 seconds may be implemented at block 218 of algorithm 210. For example, a time threshold of 1 minute or even up to 2 minutes are within the scope of the present disclosure, just to give a couple of arbitrary examples. If it is determined at block 218 that location information has not been entered manually within 30 seconds of the bed 12 being plugged in and the casters 49 locked, then algorithm 210 proceeds to block 222 to play a voice prompt reminding the caregiver that bed location data needs to be manually entered using GUI 120. The voice prompt is implemented, for example, as a sound file stored in memory 114 and played through a speaker (not shown) of bed 12. The voice prompt includes a message such as, for example, "Bed not located. Please input location." Other audio prompts are within the scope of the present disclosure at the discretion of the bed designer or programmer.

After the voice prompt is played at block 222, or substantially simultaneously with playing the voice prompt at block 222, algorithm 210 causes a bed-not-located screen 224 to be displayed on GUI 120 as shown in Fig. 4A. Screen 224 includes a close button or icon 226 that is selected if the caregiver does not wish to manually enter location data into memory 114 of bed 12 for communication via module 16 to network 18. After button 226 is selected, home screen 144 is displayed on GUI 120. Screen 224 also includes an OK icon or button 228 that is selected to navigate to further screens for manual entry of bed location data or information as will be discussed in further detail below. In the illustrative example, screen 224 also displays the text string, "BED NOT SYNCHED WITH NURSE CALL SYSTEM. INPUT BED LOCATION."

Giving caregivers a threshold period of time for entry of manual location data at block 218 of algorithm 210 is an improvement over the algorithm depicted in Fig. 11 of U.S. Patent Application Publication No. 2018/0039743 A1 in which manual entry screens are displayed in response to AC power being applied to the bed. For example, the caregiver may wish to see other information on home screen 144 regarding bed status prior to beginning the process of manually entering bed location information. Furthermore, requiring bed 12 to be plugged in to an AC outlet 170 and requiring casters 49 to be locked or braked in algorithm 210 prior to display of bed-not-located also represents an improvement over the algorithm depicted in Fig. 11 of U.S. Patent Application Publication No. 2018/0039743 A1 because the two conditions of algorithm 210 at blocks 214, 216 provides redundant assurance that the bed 12 is intended to remain at its location for an extended period of time, thereby warranting manual entry of bed location information. Still further, the algorithm of Fig. 11 of U.S. Patent Application Publication No. 2018/0039743 A1 does not include any voice prompt like the algorithm 210 of the present disclosure. The voice prompt at block 222 of algorithm 210 assures that the caregiver is alerted to the need for manual entry of bed location information if the caregiver does not otherwise notice the bed-not-located screen 224 being displayed on GUI 120, for example.

In response to OK icon 228 being selected on screen 224, algorithm 210 causes a select-the-bed-location screen 230 being displayed on GUI 120 as shown in Fig. 4A. Screen 230 also appears on GUI 120 in response to location button 150 on home screen 144 being selected by the caregiver. Furthermore, screen 230 appears on GUI 120 in response to edit icon 148 of window 142, shown in Fig. 3, being selected on home screen 144. Thus, there are three ways in which screen 230 becomes displayed on GUI 120, as follows: selection of OK button 228 on screen 224; selection of location button 150 on screen 144; and selection of edit icon 148 of window 142, if shown, on screen 144. Regardless of the manner in which the caregiver navigates to screen 230, the remaining discussion below of screen 230 and the screens of Fig. 4B is equally applicable.

Screen 230 includes a table 232 having a list of room locations that can be selected by touching the row in the table 232 corresponding to the room in which bed 12 is located. An up arrow icon 234 and a down arrow icon 236 are provided on screen 230 in the right hand portion of table 232. Icons 234, 236 are touched by the caregiver to scroll up or down, respectively, to view other room location choices that are available for selecting by the caregiver on table 232. In the illustrative example of Fig. 4A, the location of MED/SURG 2011-A is selected in table 232 as indicated by the border highlighting around the selected row in table 232. A delete icon 238 is shown to the left of the selected bed location in table 232. Beneath table 232 of screen 230 is a campus edit icon or button 240 and a unit edit button or icon 242.

At the bottom of screen 230 is a cancel button or icon 244 and a select icon or button 246. If the caregiver does not wish to make any manual location selections, the cancel button 244 is selected and GUI 120 will return to showing home screen 144. If the room location of table 232 is the desired room location and if the desired campus and unit selections appear to the right of respective buttons 240, 242, the select button 246 is selected to store the room location, campus location, and unit location information in memory 114 of control circuitry 110 of bed 12. After the bed location information is stored in memory 114, control circuitry 110 controls wireless communication module 16 of bed 12 to transmit the bed location information to one or more of servers 22, 24, 26 of the corresponding systems 23, 25, 27 of network 18 via one or more WAP's 14.

In some embodiments, module 16 of bed 12 transmits the stored location information a single time and, if an acknowledgement message is returned to module 16 of bed via one or more WAP's 14 indicating that the desired destination server(s) 22, 24, 26 has successfully received the location information from bed 12, then module does not transmit the location information subsequently unless a change is made to the location information using GUI 120 or unless bed 12 is unplugged from outlet 170 for a threshold period of time in which case algorithm 210 starts anew at block 212. In such embodiments, a bed ID such as a bed serial number or MAC address is transmitted with the bed location information. During subsequent bed status data transmissions from bed 12, the bed ID is also transmitted and is used by the receiving server(s) 22, 24, 26 to correlate or associate the bed status information with the bed location information. In other embodiments, the bed location data is transmitted by module 16 of bed along with each transmission of bed status data after the bed location information has been stored in memory 114 of control circuitry 110.

If the caregiver wishes to select a different campus location while viewing screen 230, the campus icon 240 is selected on screen 230 of Fig. 4A and algorithm 210 proceeds to display a select-the-campus-location screen 248 on GUI 120 as shown in Fig. 4B. The line labeled with circle A shows the screen flow of algorithm 210 in this regard. Screen 248 includes a table 250 having a list of campus locations that can be selected by touching the row in the table 250 corresponding to the campus at which bed 12 is located. Up and down arrow icons 234, 236 are provided at the right hand portion of table 250 and are touched to scroll up or down, respectively, to view other campus location choices that are available for selecting by the caregiver on table 250. In the illustrative example of Fig. 4B, the campus of ST. ELIZABETH SOUTH is selected in table 250 as indicated by the border highlighting around the selected row in table 250.

Delete icon 238 is shown to the left of the selected campus location in table 250. Beneath table 250 of screen 248 is campus edit icon or button 240 and a unit edit button or icon 242. At the bottom of screen 248 is cancel button or icon 244 and select icon or button 246. Buttons 238, 240, 242, 244, 246 on screen 248 function in the same manner as these same buttons 238, 240, 242, 244, 246 function on screen 230. For example, if the caregiver does not wish to make any manual location selections on screen 248, the cancel button 244 is selected and GUI 120 will return to showing home screen 144 or, in some embodiments, screen 230. If the campus location of table 250 is the desired campus location and if the desired unit selection also appears to the right of button 242, the select button 246 is selected to store the campus location and unit location in memory 114 of control circuitry 110 of bed 12 for eventual transmission by module 16 as described above.

If the caregiver wishes to select a different unit location while viewing screen 230 or while viewing screen 248, the unit icon 242 is selected on screen 230 of Fig. 4A or screen 248 of Fig. 4B, as the case may be, and algorithm 210 proceeds to display a select-the-unit-location screen 252 on GUI 120 as shown in Fig. 4B. The line labeled with circle B shows the screen flow of algorithm 210 in this regard in connection with selection of button 242 on screen 230. Screen 252 also appears on GUI 120 in response to the caregiver selecting button 246 on screen 248. In either case, screen 252 includes a table 254 having a list of unit locations that can be selected by touching the row in the table 254 corresponding to the unit at which bed 12 is located. Up and down arrow icons 234, 236 are provided at the right hand portion of table 254 and are touched to scroll up or down, respectively, to view other unit location choices that are available for selecting by the caregiver on table 254. In the illustrative example of Fig. 4B, the unit of WEST TOWER is selected in table 254 as indicated by the border highlighting around the selected row in table 254.

Delete icon 238 is shown to the left of the selected unit location in table 254. Beneath table 254 of screen 252 is campus edit icon or button 240 and unit edit button or icon 242. At the bottom of screen 252 is cancel button or icon 244 and select icon or button 246. Buttons 238, 240, 242, 244, 246 on screen 252 function in the same manner as these same buttons 238, 240, 242, 244, 246 function on screens 230, 248. For example, if the caregiver does not wish to make any manual location selections on screen 252, the cancel button 244 is selected and GUI 120 will return to showing home screen 144 or, in some embodiments, return to showing screen 230. If the unit location of table 254 is the desired unit location and if the desired campus selection also appears to the right of button 240, the select button 246 of screen 252 is selected to store the campus location and unit location in memory 114 of control circuitry 110 of bed 12 for eventual transmission by module 16 as described above.

As noted above with regard to screen 230, if the room location of table 232 is the desired room location and if the desired campus and unit selections appear to the right of respective buttons 240, 242, the select button 246 is selected to store the room location, campus location, and unit location information in memory 114 of control circuitry 110 of bed 12. Selection of button 246 on screen 230 of Fig. 4A results in a modified start-up screen 140' appearing on GUI 120 as shown in Fig. 4B. The line labeled with circle C shows the screen flow of algorithm 210 in this regard in connection with selection of button 246 on screen 230. On screen 140', window 142 now shows text string 146 with the selected room location information, MED/SURG 2011-A in the illustrative example, that was chosen using table 232 of screen 230. Window 142 appears on screen 140' for a threshold period of time, such as 5 to 30 seconds, and then disappears as was described above.

While window 142 appears on screen 140', the caregiver has the option of selecting edit icon 148 to further edit the bed location information if desired. In response to selection of button 148 on screen 140', algorithm 210 causes a current-bed-location screen 256 to appear on GUI 120 as shown in Fig. 4B. Screen 256 includes a table 258 that indicates with border highlighting the current bed location that was selected previously. The caregiver can then make another selection on table 258 to change the room location information. Scroll arrows 234, 236 are provided in table 258 for use by the caregiver to scroll other room location options as noted above. Buttons 240, 242, 244, 246 appear on screen 256 and function in the same manner as described above. For example, selection of button 240 results in navigation to screen 248, selection of button 242 results in navigation to screen 252, selection of button 244 results in navigation back to screen 140', and selection of button 246 results in navigation back to screen 140' but with the new room location information manually selected on table 258 appearing as text string 146 in window 142.

In response to button 238 geing selected on screen 256 of Fig. 4B, algorithm 210 causes screen 230 to, once again, be shown on GUI 120. The description above of screen 230 shown in Fig. 4A is equally applicable to screen 230 shown in Fig. 4B. Selection of button 238 on screen 230, be it the one shown in Fig. 4A or the one shown in Fig. 4B, results in a location delete screen 260 appearing on GUI 120. Thus, button 238 is selected when the caregiver wishes to delete a room location from even appearing on table 232. Screen 260 includes the following textual information: "ARE YOU SURE YOU WANT TO DELETE THIS LOCATION? RESTORING A LOCATION MUST BE DONE IN NURSE CALL SYSTEM." Screen 260 includes a yes button 262 that is selected if the caregiver does, indeed, wish to delete the selected bed location information from table 232 altogether. Screen 260 also includes a no button 264 that is selected to abort the deletion of the selected of the bed location information selected on table 232.

After either of buttons 262, 264 is selected on screen 260, algorithm 210 returns back to screen 230 so that the caregiver can, if desired, select the bed location information from among the remaining information on table 232 and then select icon 246, or the user can select icon 244 and return to screen 140' or screen 144 as the case may be. Button 238 of screen 248 and button 238 of screen 252 operate in a substantially similar manner as button 238 of screen 230 except that selected campus location information or selected unit location information, as the case may be, are deleted from the respective tables 250, 254 by navigating to screen 260 and selecting the yes button 262.

Although certain illustrative examples have been described in detail above, variations and modifications exist.

## Claims

1. A patient bed (12) comprising
a frame (40) configured to support a patient,
circuitry (110) carried by the frame (40), and
a graphical user interface (GUI) (120) carried by the frame (40) and coupled to the circuitry (110), the graphical user interface (120) displaying at least one user interface screen that is used by a caregiver to manually enter location data indicative of a location in a healthcare facility at which the patient bed is located, the circuitry being configured to transmit the location data entered by the caregiver and a bed identification (ID) from the bed, wherein the circuitry commands the GUI to display a first screen of the at least one interface screen that is used by the caregiver to manually enter location data, the first screen being displayed after a threshold period of time elapses subsequent to a power plug of the circuitry being plugged into an alternating current (AC) outlet of the healthcare facility and subsequent to casters of the frame being braked.

2. The patient bed (12) of claim 1, wherein the circuitry (110) is configured to play a voice prompt to remind the caregiver to manually enter the location data after the threshold period of time elapses.

3. The patient bed (12) of either claim 1 or claim 2, wherein the first screen appearing on the GUI (120) after the threshold period of time elapses includes a button that is selectable to initiate manual entry of the location data.

4. A patient bed (12) comprising
a frame (40) configured to support a patient,
circuitry (110) carried by the frame (40), and
a graphical user interface (GUI) (120) carried by the frame (40) and coupled to the circuitry (110), the graphical user interface (120) displaying at least one user interface screen that is used by a caregiver to manually enter location data indicative of a location in a healthcare facility at which the patient bed is located, the circuitry being configured to transmit the location data entered by the caregiver and a bed identification (ID) from the bed, wherein the circuitry is configured to play a voice prompt to remind the caregiver to manually enter the location data after a threshold period of time elapses subsequent to a power plug of the circuitry being plugged into an alternating current (AC) outlet of the healthcare facility and subsequent to casters of the frame being braked.

5. The patient bed (12) of claim 4, wherein the circuitry also commands the GUI (120) to display a first screen of at least one interface screen that is used by the caregiver to manually enter location data after the threshold period of time elapses.

6. The patient bed (12) of either claim 4 or claim 5, wherein the first screen appearing on the GUI (120) includes a button that is selectable to initiate manual entry of the location data.

7. The patient bed (12) of any preceding claim, wherein the GUI (120) includes a change location button that is selectable by the caregiver to initiate a change of the location data prior to the threshold period of time elapsing.

8. The patient bed (12) of any one of claims 3, 6 or 7, wherein a room menu screen appears on the GUI (120) in response to the button being selected on the first screen, the room menu screen permitting the caregiver to select a room number from a list of room numbers.

9. The patient bed (12) of claim 8, wherein a campus icon and a unit icon appears on the GUI (120) in response to the button being selected on the first screen, the campus icon being selectable to cause a campus menu list being displayed on the GUI (120) and the unit icon being selectable to cause a unit menu list being displayed on the GUI (120).

10. The patient bed (12) of any preceding claim, wherein the circuitry (110) includes a wireless communication module (16) configured to wirelessly transmit the location data and the bed ID to a wireless access point (14) for delivery to at least one remote computer for purposes of making a bed-to-room association.

11. The patient bed (12) of claim 10, wherein the wireless communication module (14) also transmits bed status data from the bed.

12. The patient bed (12) of any preceding claim, wherein the location data comprises a room number.

## Patentansprüche

1. Patientenbett (12), das Folgendes aufweist:
ein Gestell (40), das zum Lagern eines Patienten konfiguriert ist,
eine Schaltungsanordnung (110), die von dem Gestell (40) getragen wird, und
eine graphische Benutzeroberfläche (GUI) (120), die von dem Gestell (40) getragen wird und mit der Schaltungsanordnung (110) gekoppelt ist, wobei die graphische Benutzeroberfläche (120) wenigstens einen Benutzeroberflächenbildschirm anzeigt, der von einer Pflegeperson zum manuellen Eingeben von Standortdaten verwendet wird, die einen Standort in einer Gesundheitseinrichtung bezeichnen, an dem sich das Patientenbett befindet, wobei die Schaltungsanordnung zum Übertragen der von der Pflegeperson eingegebenen Standortdaten und einer Bettkennzeichnung (ID) von dem Bett konfiguriert ist, wobei die Schaltungsanordnung der GUI das Anzeigen eines ersten Bildschirms des wenigstens einen Benutzeroberflächenbildschirms befiehlt, der von der Pflegeperson zum manuellen Eingeben von Standortdaten verwendet wird, wobei der erste Bildschirm nach Verstreichen einer Schwellenzeitspanne im Anschluss an das Einstecken eines Netzsteckers der Schaltungsanordnung in eine Wechselstrom- (AC) -steckdose der Gesundheitseinrichtung und anschließendes Bremsen der Rollen des Gestells angezeigt wird.

2. Patientenbett (12) nach Anspruch 1, wobei die Schaltungsanordnung (110) zum Abspielen einer
Sprachaufforderung zum Erinnern der Pflegeperson an das manuelle Eingeben der Standortdaten nach Verstreichen der Schwellenzeitspanne konfiguriert ist.

3. Patientenbett (12) nach Anspruch 1 oder Anspruch 2, wobei der erste Bildschirm, der nach Verstreichen der Schwellenzeitspanne auf der GUI (120) erscheint, eine Schaltfläche beinhaltet, die zum Einleiten einer manuellen Eingabe der Standortdaten auswählbar ist.

4. Patientenbett (12), das Folgendes aufweist:
ein Gestell (40), das zum Lagern eines Patienten konfiguriert ist,
ein Gestell (40), das zum Lagern eines Patienten konfiguriert ist,
eine Schaltungsanordnung (110), die von dem Gestell (40) getragen wird, und
eine graphische Benutzeroberfläche (GUI) (120), die von dem Gestell (40) getragen wird und mit der Schaltungsanordnung (110) gekoppelt ist, die graphische Benutzeroberfläche (120) dabei wenigstens einen Benutzeroberflächenbildschirm anzeigt, der von einer Pflegeperson zum manuellen Eingeben von Standortdaten verwendet wird, die einen Standort in einer Gesundheitseinrichtung bezeichnen, an dem sich das Patientenbett befindet, die Schaltungsanordnung dabei zum Übertragen der von der Pflegeperson eingegebenen Standortdaten und einen Bett-Kennzeichnung (ID) von dem Bett konfiguriert ist, wobei die Schaltungsanordnung zum Abspielen einer Sprachaufforderung zum Erinnern der Pflegeperson an das manuelle Eingeben der Standortdaten nach Verstreichen einer Schwellenzeitspanne im Anschluss an das Einstecken eines Netzsteckers der Schaltungsanordnung in eine Wechselstrom-(AC) -steckdose der Gesundheitseinrichtung und im Anschluss an das Bremsen von Rollen des Gestells konfiguriert ist.

5. Patientenbett (12) nach Anspruch 4, wobei die Schaltungsanordnung auch der GUI (120) das Anzeigen eines ersten Bildschirms des wenigstens einen Benutzeroberflächenbildschirms befiehlt, der von der Pflegeperson zum manuellen Eingeben von Standortdaten nach Verstreichen der Schwellenzeitspanne verwendet wird.

6. Patientenbett (12) nach Anspruch 4 oder Anspruch 5, wobei der erste auf der GUI (120) erscheinende Bildschirm eine Schaltfläche beinhaltet, die zum Einleiten der manuellen Eingabe der Standortdaten auswählbar ist.

7. Patientenbett (12) nach einem der vorhergehenden Ansprüche, wobei die GUI (120) eine Standort-ändern-Schaltfläche beinhaltet, die von der Pflegeperson zum Einleiten einer Änderung der Standortdaten vor Verstreichen der Schwellenzeitspanne auswählbar ist.

8. Patientenbett (12) nach einem der Ansprüche 3, 6 oder 7, wobei auf der GUI (120) als Reaktion auf das Auswählen der Schaltfläche auf dem ersten Bildschirm ein Zimmermenübildschirm erscheint, der Zimmermenübildschirm dabei zulässt, dass die Pflegeperson eine Zimmernummer aus einer Zimmernummernliste auswählt.

9. Patientenbett (12) nach Anspruch 8, wobei auf der GUI (120) als Reaktion auf das Auswählen der Schaltfläche auf dem ersten Bildschirm ein Geländesymbol und ein Einheitssymbol erscheinen, wobei das Geländesymbol auswählbar ist, um zu veranlassen, dass auf der GUI (120) angezeigt wird, und das Einheitssymbol auswählbar ist, um zu veranlassen, dass auf der GUI (120) eine Einheitsmenüliste angezeigt wird.

10. Patientenbett (12) nach einem der vorhergehenden Ansprüche, wobei die Schaltungsanordnung (110) ein Wireless-Kommunikationsmodul (16) beinhaltet, das zum drahtlosen Übertragen der Standortdaten und der Bettkennzeichnung an einen Wireless Access Point (14) zur Lieferung an wenigstens einen fernen Computer zum Zweck der Herstellung einer Bett-Zimmer-Zuordnung konfiguriert ist.

11. Patientenbett (12) nach Anspruch 10, wobei das Wireless-Kommunikationsmodul (14) auch Bettstatusdaten von dem Bett überträgt.

12. Patientenbett (12) nach einem der vorhergehenden Ansprüche, wobei die Standortdaten eine Zimmernummer aufweisen.

## Revendications

1. Lit de patient (12) comprenant :
un cadre (40) configuré pour soutenir un patient,
des circuits (110) portés par le cadre (40), et
une interface utilisateur graphique (IUG) (120) portée par le cadre (40) et couplée aux circuits (110), l'interface utilisateur graphique (120) affichant au moins un écran d'interface utilisateur qui est utilisé par un soignant pour entrer manuellement des données d'emplacement indicatives d'un emplacement dans un établissement de soins de santé auquel le lit de patient est situé, les circuits étant configurés pour transmettre les données d'emplacement entrées par le soignant et une identification de lit (ID) du lit, dans lequel les circuits commandent à l'IUG d'afficher un premier écran du au moins un écran d'interface qui est utilisé par le soignant pour entrer manuellement les données d'emplacement, le premier écran étant affiché après qu'une période de temps seuil s'est écoulée après qu'une fiche d'alimentation des circuits a été branchée dans une prise en courant alternatif (CA) de l'établissement de soins de santé et après que les roulettes du cadre ont été freinées.

2. Lit de patient (12) selon la revendication 1, dans lequel les circuits (110) sont configurés pour diffuser une invite vocale afin de rappeler au soignant d'entrer manuellement les données d'emplacement après que la période de temps seuil s'est écoulée.

3. Lit de patient (12) selon la revendication 1 ou la revendication 2, dans lequel le premier écran apparaissant sur l'IUG (120) après que la première période de temps seuil s'est écoulée comprend un bouton qui est sélectionnable pour déclencher l'entrée manuelle des données d'emplacement.

4. Lit de patient (12) comprenant
un cadre (40) configuré pour soutenir un patient,
des circuits (110) portés par le cadre (40), et
une interface utilisateur graphique (IUG) (120) portée par le cadre (40) et couplée aux circuits (110), l'interface utilisateur graphique (120) affichant au moins un écran d'interface utilisateur qui est utilisé par un soignant pour entrer manuellement des données d'emplacement indicatives d'un emplacement dans un établissement de soins de santé auquel le lit de patient est situé, les circuits étant configurés pour transmettre les données d'emplacement entrées par le soignant et une identification de lit (ID) du lit, dans lequel les circuits sont configurés pour diffuser une invite vocale afin de rappeler au soignant d'entrer manuellement les données d'emplacement après qu'une période de temps seuil s'est écoulée après qu'une fiche d'alimentation des circuits a été branchée dans une prise en courant alternatif (CA) de l'établissement de soins de santé et après que les roulettes du cadre ont été freinées.

5. Lit de patient (12) selon la revendication 4, dans lequel les circuits commandent aussi à l'IUG (120) d'afficher un premier écran du au moins un écran d'interface qui est utilisé par le soignant pour entrer manuellement les données d'emplacement après que la période de temps seuil s'est écoulée.

6. Lit de patient (12) selon la revendication 4 ou la revendication 5, dans lequel le premier écran apparaissant sur l'IUG (120) comprend un bouton qui est sélectionnable pour déclencher l'entrée manuelle des données d'emplacement.

7. Lit de patient (12) selon l'une quelconque des revendications précédentes, dans lequel l'IUG (120) comprend un bouton de changement d'emplacement qui est sélectionnable par le soignant pour déclencher un changement des données d'emplacement avant que la période de temps seuil ne s'écoule.

8. Lit de patient (12) selon l'une quelconque des revendications 3, 6 ou 7, dans lequel un écran de menu de chambres apparait sur l'IUG (120) en réponse au bouton étant sélectionné sur le premier écran, l'écran de menu de chambres permettant au soignant de sélectionner un numéro de chambre d'une liste de numéros de chambre.

9. Lit de patient (12) selon la revendication 8, dans lequel une icône de campus et une icône d'unités apparaissent sur l'IUG (120) en réponse au bouton étant sélectionné sur le premier écran, l'icône de campus étant sélectionnable pour faire qu'une liste de menu de campus soit affichée sur l'IUG (120) et l'icône d'unités étant sélectionnable pour faire qu'une liste de menu d'unités soit affichée sur l'IUG (120).

10. Lit de patient (12) selon l'une quelconque des revendications précédentes, dans lequel les circuits (110) comprennent un module de communication sans fil (16) configuré pour transmettre sans fil les données d'emplacement et l'ID du lit à un point d'accès sans fil (14) pour remise à au moins un ordinateur à distance dans le but de faire une association de lit à chambre.

11. Lit de patient (12) selon la revendication 10, dans lequel le module de communication sans fil (14) transmet aussi des données d'état de lit du lit.

12. Lit de patient (12) selon l'une quelconque des revendications précédentes, dans lequel les données d'emplacement comprennent un numéro de chambre.
